# EUROPEAN PATENT SPECIFICATION

(11) **EP 3 424 412 B1**
(45) Date of publication and mention of the grant of the patent: **29.03.2023**
(21) Application number: 18181530.9
(22) Date of filing: 03.07.2018
(51) Int. Cl.: A61B 5/00, A61B 5/145, A61B 5/1495

(54) **MEASURING APPARATUS, MEASURING PROGRAM AND MEASURING METHOD**
MESSVORRICHTUNG, MESSPROGRAMM UND MESSVERFAHREN
APPAREIL DE MESURE, PROGRAMME DE MESURE ET PROCÉDÉ DE MESURE

(30) Priority: 04.07.2017 JP 2017131323; 28.06.2018 JP 2018123516
(43) Date of publication of application: 09.01.2019
(73) Proprietor: ARKRAY, Inc., Kyoto-shi, Kyoto 601-8045 (JP)
(72) Inventor: SHIMIZU, Takeshi, Kyoto-shi, Kyoto 602-0008 (JP)
(74) Representative: MacDougall, Alan John Shaw

(56) References cited:
- WO-A1-2017/035021
- US-A1- 2005 203 364
- US-A1- 2011 184 268
- US-A1- 2015 130 633

## Description

### FIELD

An embodiment pertain to a measuring apparatus, a measuring program and a measuring method.

### BACKGROUND

A variety of blood glucose level measuring apparatuses for measuring a self blood glucose level (a glucose concentration value in blood) are currently put on sale in the market. Known as the blood glucose level measuring apparatus are, e.g., a Self-Monitoring of Blood Glucose (SMBG) apparatus for self-monitoring (measuring) of the blood glucose and a Continuous Glucose Monitoring (CGM) apparatus for continuous glucose monitoring (measuring). The SMBG apparatus measures the blood glucose level in a way that attaches blood extracted from a finger tip by using a needling tool onto a test piece fitted to measurement equipment. The CGM apparatus continuously measures a glucose concentration in interstitial fluid by subcutaneously indwelling a micro sensor including electrodes and enzyme reacting to the glucose. In the CGM apparatus, a sensor is indwelled subcutaneously over a period as long as several days through several weeks, and a blood glucose level is consecutively measured at an interval of several tens of seconds through several minutes. A review about where the CGM apparatus is set on a body of a patient is underway in consideration for relieving a pain and other equivalent symptoms of the patient. It is known to dispose the sensor on an abdominal region, a brachial region, a femoral region and other equivalent regions of a measurement examinee (e.g., Patent document 1).

[Patent document 1] Japanese Patent No. 5904500

WO2017/035021 describes an infusion system in which performance metrics of an in vivo sensor are monitored and feedback is provided, which may include recommended sensor site locations for rotation or replacement of the sensing arrangement determined based on historical data associated with the user for different sensor site locations. The current sensor site may be automatically identified or detected when sufficient historical site data exists after an initial setup or training phase.

US2011/184268 describes a method and device for providing calibration in analyte monitoring systems. In some embodiments, the user can input the testing location to improve sensor calibration.

US2015/130633 describes a system and method for tracking sensor insertion locations in a continuous analyte monitoring system. Data gathered from sensors can be used to provide a user with suggested rotation of insertion location, and a suggested next insertion location based upon past sensor accuracy and/or sensor session length at that location.

### SUMMARY

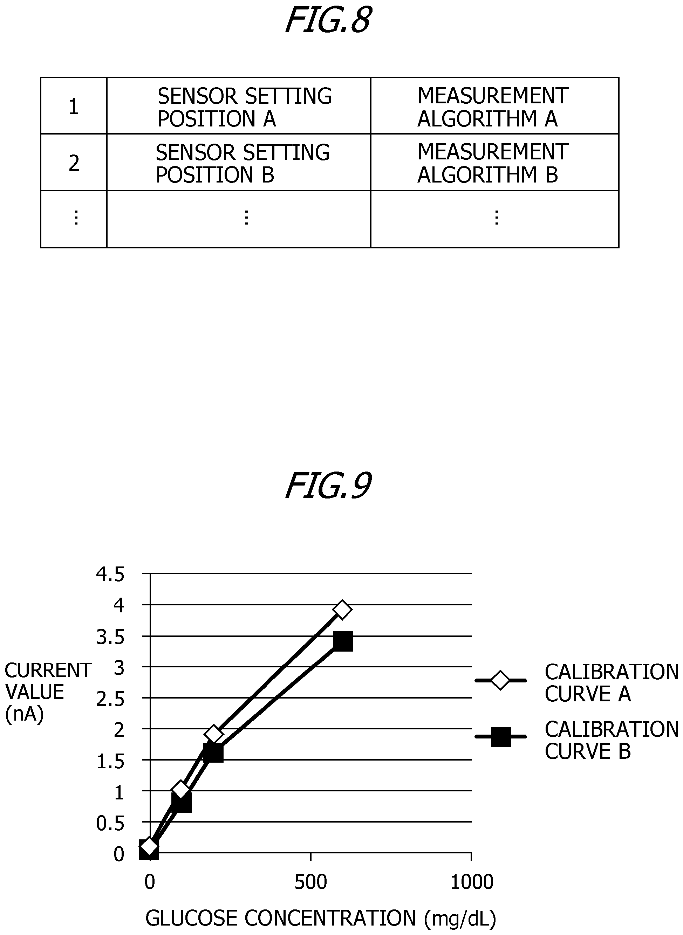

For example, the CGM apparatus is set on the region instanced by the brachial region, the abdominal region and a gluteal region of the patient, and measures a glucose concentration in an interstitial fluid. A time lag exists between the blood glucose level and the glucose concentration value in the interstitial fluid. In other words, a fixed period of time is taken till the glucose concentration value in the interstitial fluid reflects the blood glucose level. The time taken till the glucose concentration value in the interstitial fluid reflects the blood glucose level differs depending on a position where the CGM apparatus is set on the patient. For instance, when a measured current value is converted into the glucose concentration value by using a calibration curve, as illustrated in FIG. 9, the calibration curve differs depending on the setting position of the CGM apparatus. A calibration curve A in FIG. 9 is a calibration curve when the CGM apparatus is set on, e.g., the brachial region of the patient, while a calibration curve B in FIG. 9 is a calibration curve when the CGM apparatus is set on, e.g., the abdominal region of the patient. However, the CGM apparatus is set in an arbitrary position of the patient, and the CGM apparatus makes the measurement without taking account of the setting position of the CGM apparatus, in which case measurement accuracy of the CGM apparatus decreases. Embodiments, which are made in view of such circumstances, aim at improving measurement accuracy of a concentration value of a specified substance in a sample.

According to an aspect of the embodiment, a measuring apparatus includes: a sensor configured to be indwelled in vivo of a user; an identifying unit configured to identify a setting position of the sensor; a calculation unit configured to calculate a concentration of a specified substance contained in a sample, based on a signal given from the sensor; and a storage unit configured to store plural pieces of arithmetic information, wherein the calculation unit selects one of the plural pieces of arithmetic information from the storage unit, based on the setting position of the sensor identified by the identifying unit and calculates the concentration of the specified substance contained in the sample by using the selected arithmetic information; wherein the apparatus comprises a detection sensor configured to be attached to the user to detect an activity factor of the user, and the identifying unit is configured to identify the setting position of the sensor based on the activity factor of the user acquired from the detection sensor.

According to the embodiment, the arithmetic information associated with the setting position of the sensor is selected from within the plural pieces of arithmetic information. The concentration value of the specified substance contained in the sample is calculated based on the signal given from the sensor by using the selected arithmetic information. The concentration value of the specified substance contained in the sample is calculated based on the signal given from the sensor by using the arithmetic information selected from within the plural pieces of arithmetic information each being different for every setting position of the sensor, thereby enabling improvement of the measurement accuracy of the concentration value of the specified substance contained in the sample.

In the measuring apparatus, the plural pieces of arithmetic information contain plural items of calibration curve data, and the calculation unit selects one of the plural items of calibration curve data from the storage unit, based on the setting position of the sensor identified by the identifying unit and calculates the concentration of the specified substance contained in the sample by using the selected calibration curve data. In the measuring apparatus, the plural pieces of arithmetic information contain plural measurement algorithms, and the calculation unit selects one of the plural measurement algorithms from the storage unit, based on the setting position of the sensor identified by the identifying unit and calculates the concentration of the specified substance contained in the sample by using the selected measurement algorithms.

In the measuring apparatus, the activity factor may comprise a motion quantity of a user, an attitude of the sensor, a body temperature of the user, a pulse rate, a heart rate or a pulse wave in respect of the user, and identifies the sensor setting position, based on the pulse rate, the heart rate or the pulse wave in respect of the user, or a blood pressure value in respect of the user. In the measuring apparatus, the sensor consecutively measures the signals. In the measuring apparatus, the sample is interstitial fluid.

The aspect described above may be attained in a way that causes a program to be run by a computer. To be specific, the aspect described above may be specified as a program to be run by the computer, or as a computer readable recording medium on which the program is recorded. The aspect described above may also be specified as a method executed by the computer. The aspect described above may further be specified as a system including the measuring apparatus.

According to the embodiment, it is feasible to improve the measurement accuracy of the concentration value of the specified substance in the sample.

### BRIEF DESCRIPTION OF DRAWINGS

FIG. 1 is a view of a configuration of a measuring system according to a first embodiment;
FIG. 2 is a block diagram of a configuration of a transmitting apparatus according to the first embodiment;
FIG. 3 is a block diagram of a configuration of a receiving apparatus according to the first embodiment;
FIG. 4 is diagram illustrating a value of activity factor data when a measurement sensor is indwelled subcutaneously in a brachial region of a user, and a value of the activity factor data when the measurement sensor is indwelled subcutaneously in an abdominal region of the user;
FIG. 5 is a table illustrating one example of plural items of calibration curve data pre-stored in a storage unit;
FIG. 6 is a flowchart illustrating one example of a measurement process according to the first embodiment;
FIG. 7 is a flowchart illustrating one example of the measurement process according to a second embodiment;
FIG. 8 is a table illustrating one example of plural measurement algorithms pre-stored in the storage unit;
FIG. 9 is a graphic chart illustrating one example of calibration curves;
FIG. 10 is a view of a configuration of a measuring system according to a third embodiment;
FIG. 11 is a block diagram of a configuration of a dosage apparatus according to the third embodiment;
FIG. 12 is a flowchart illustrating an example of a measurement process according to the third embodiment; and
FIG. 13 is a flowchart illustrating an example of the measurement process according to the third embodiment.

### DESCRIPTION OF EMBODIMENT

Embodiments will hereinafter be described with reference to the drawings. The following respective embodiments are exemplifications, and the present invention is not limited to configurations of the embodiments given below.

### <First Embodiment>

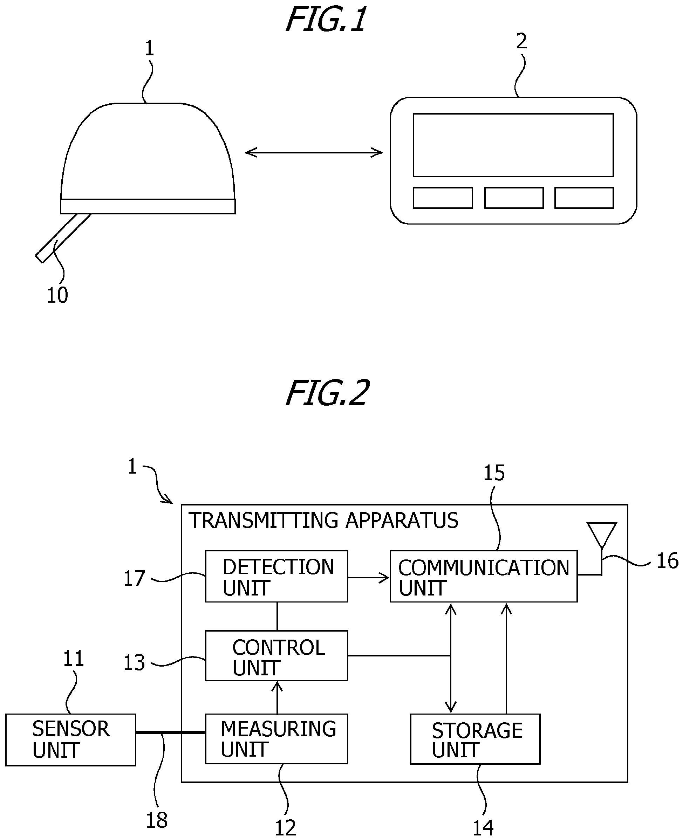

FIG. 1 is a diagram of a configuration of a measuring system according to a first embodiment. The measuring system illustrated in FIG. 1 includes a transmitting apparatus 1 and a receiving apparatus 2. The transmitting apparatus 1 consecutively measures a concentration of a specified substance (measurement target substance) in a sample in vivo, and transmits a measurement result to the receiving apparatus 2. The transmitting apparatus 1 may be used by being attached to regions instanced by a brachial region, an abdominal region, a gluteal region and a leg region of a user (patient). The transmitting apparatus 1 may also be a Continuous Glucose Monitoring (CGM) apparatus for performing continuous glucose monitoring (measurement). A body fluid instanced by an interstitial fluid is given as the sample in vivo. The specified substance is exemplified by glucose contained in the interstitial fluid. The specified substance may also be a substance other than glucose.

The receiving apparatus 2 receives a measurement result from the transmitting apparatus 1. The receiving apparatus 2 performs wireless data communications with the transmitting apparatus 1. The receiving apparatus 2 may also perform wired data communications with the transmitting apparatus 1. The transmitting apparatus 1 and the receiving apparatus 2 are configured as separate equipments in the first embodiment, and may also be configured integrally.

### transmitting Apparatus>

The transmitting apparatus 1 includes a measurement sensor 10 is used by being implanted into a subcutaneous region of the user. The transmitting apparatus 1 is pasted to a skin of the user by an adhesive tape and other equivalent materials, or is attached to a belt and other equivalent articles, thereby being fitted to the user. The measurement sensor 10 is an electrochemical sensor that measures a specified component (e.g., a concentration of the specified substance) in the sample by utilizing electrochemical reaction. The measurement sensor 10 is indwelled subcutaneously over a consecutive measurement period as long as, e.g., several days through several weeks, and the transmitting apparatus 1 consecutively measures the glucose concentration in the interstitial fluid. "Being consecutive" connotes that the transmitting apparatus 1 continuously measures the glucose concentration in a state of the measurement sensor 10 being subcutaneously indwelled, and encompasses such a mode that the transmitting apparatus 1 measures the glucose concentration at an interval of predetermined time. A measurement frequency of the glucose concentration may be arbitrarily set with respect to the transmitting apparatus 1. For example, the measurement frequency of the glucose concentration may also be set with respect to the transmitting apparatus 1 so that the transmitting apparatus 1 measures the glucose concentration at a frequency of once every several ten seconds through several minutes.

FIG. 2 is a block diagram of a configuration of the transmitting apparatus 1 according to the first embodiment. The transmitting apparatus 1 is a transmitter that transmits various items of data to the receiving apparatus 2. The transmitting apparatus 1 includes a sensor unit 11, a measurement unit 12, a control unit (arithmetic unit) 13, a storage unit 14, a communication unit 15, an antenna 16, and a detection sensor 17. The sensor unit 11 has glucose oxidoreductase instanced by glucose oxidase (GOD) and glucose dehydrogenase (GDH), and a plurality of electrodes, i.e., a working electrode, a counter electrode, a reference electrode and other equivalent electrodes. The sensor unit 11 is provided on the side of a tip of the measurement sensor 10 illustrated in FIG. 1. The sensor unit 11 is electrically connected to the measurement unit 12 via a wire 18.

The measurement unit 12 is a circuit to measure a signal value (e.g., a response current value) by applying a voltage to the sensor unit 11. When the voltage is applied to between the electrodes (between the working electrode and the counter electrode, or between the working electrode and the reference electrode) of the sensor unit 11, the sensor unit 11 outputs a response current value corresponding to the glucose concentration in the body fluid. The measurement unit 12 measures the response current value outputted from the sensor unit 11 in a way that controls the voltage to be applied to between the electrodes of the sensor unit 11. When the voltage is applied to between the electrodes of the sensor unit 11, the glucose in the body fluid is oxidized by the oxidoreductase, and electrons being thereby extracted are supplied to the working electrode. The measurement unit 12 measures, as the response current value, a quantity of electric charges of the electrons supplied to the working electrode. The measurement unit 12 may convert the response current value into a response voltage value, and may measure, as the response voltage value, the quantity of electric charges of the electrons supplied to the working electrode. The following discussion will deal with a case that the measurement unit 12 measures the response current value. The response current value measured by the measurement unit 12 is sent to the control unit 13.

The control unit 13 controls the measurement unit 12, the storage unit 14, the communication unit 15, and the detection sensor 17. The control unit 13, the storage unit 14 and the communication unit 15 may be attained by: computers each including a Central Processing Unit (CPU), a Random Access Memory (RAM), a Read Only Memory (ROM) and other equivalent hardware components that are provided in the transmitting apparatus 1; respective apparatuses; and programs and other equivalent software components running on the computer. The CPU is also called a processor. It does not mean that the CPU is limited to the single processor, and the CPU may, however, take a multi-processor configuration.

The control unit 13 stores the response current value in the storage unit 14, and sends the response current value to the communication unit 15. The communication unit 15 transmits the response current value to the receiving apparatus 2 via the antenna 16. The communication unit 15 may send the response current value transmitted from the control unit 13 to the receiving apparatus 2, and may also send the response current value stored in the storage unit 14 to the receiving apparatus 2.

The detection sensor 17 detects an activity factor of the user attached with the transmitting apparatus 1, and outputs a signal corresponding to the activity factor of the user. The detection sensor 17 detects a motion quantity and an attitude of the measurement sensor 10, and outputs signals corresponding to the motion quantity and the attitude of the measurement sensor 10. The transmitting apparatus 1 is attached to the user, and hence the detection sensor 17 is set on the user. The detection sensor 17 has at least one of a motion sensor, an attitude sensor and a vital sensor. The motion sensor is an activity sensor to detect a quantity of motion of the user attached with the transmitting apparatus 1, and, e.g., an acceleration sensor is given as the motion sensor. The motion sensor may also detect a motion quantity of the measurement sensor 10. The attitude sensor is an activity sensor to detect an attitude (direction) of a user's region attached with the transmitting apparatus 1, and, e.g., a gyro sensor is given as the attitude sensor. The attitude sensor may also detect an attitude of the measurement sensor 10. The vital sensor is an activity sensor to measure vital data of the user attached with the transmitting apparatus 1, and, e.g., each of a temperature sensor, a pulse sensor, a heartbeat sensor, a pulse wave sensor and a blood pressure sensor is given as the vital sensor. The temperature sensor measures a body temperature of the user attached with the transmitting apparatus 1. The pulse sensor measures a pulse rate of the user attached with the transmitting apparatus 1. The heartbeat sensor measures a heart rate (heartbeat rate) of the user attached with the transmitting apparatus 1. The pulse wave sensor measures pulse waves of the user attached with the transmitting apparatus 1. The blood pressure sensor measures a blood pressure level of the user attached with the transmitting apparatus 1.

The user's activity factor detected by the detection sensor 17 is sent as activity factor data to the communication unit 15. The communication unit 15 transmits the activity factor data to the receiving apparatus 2 via the antenna 16. The activity factor data includes at least one of motion quantity data (acceleration data), attitude data (angular velocity data and angular acceleration data), body temperature data, pulse rate data, heart rate data, pulse wave data and blood pressure level data.

### <Receiving Apparatus>

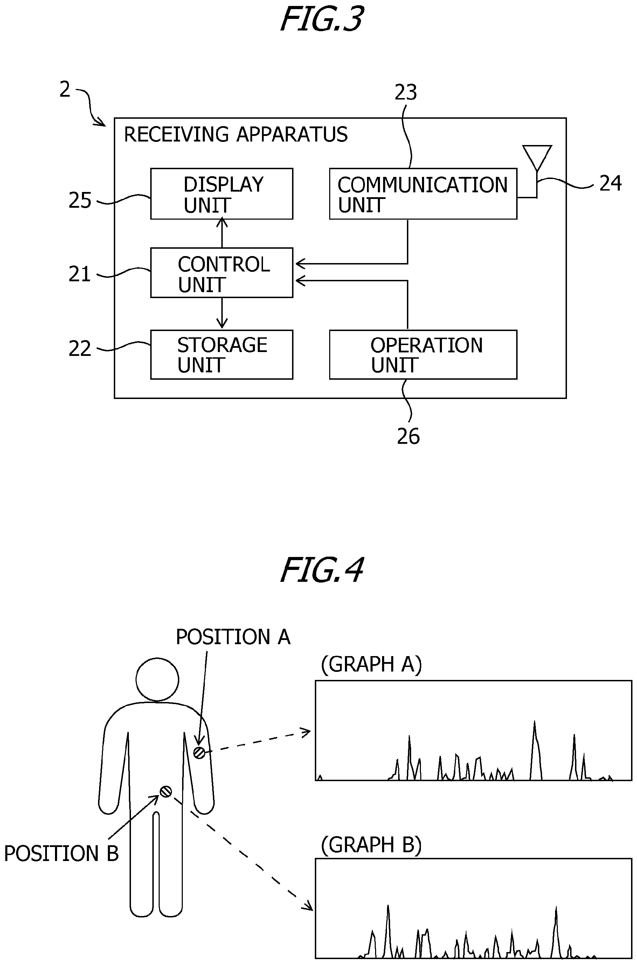

FIG. 3 is a block diagram illustrating a configuration of the receiving apparatus 2 according to the first embodiment. The receiving apparatus 2 receives the various items of data from the transmitting apparatus 1, and displays the received data. The receiving apparatus 2 includes a control unit (arithmetic unit) 21, a storage unit 22, a communication unit 23, an antenna 24, a display unit 25, and an operation unit 26. The control unit 21 controls the storage unit 22, the communication unit 23, and the display unit 25. The control unit 21, the storage unit 22, and the communication unit 23 may be attained by: computers each including the CPU, the RAM, the ROM and other equivalent hardware components that are provided in the receiving apparatus 2; respective apparatuses; and programs and other equivalent software components running on the computer.

The display unit 25 has a display and displays various types of information and messages on this display. The display unit 25 displays a measurement result and an error on the display, and also displays operation procedures, operation statuses and other equivalent items when setting is done. The display of the display unit 25 is exemplified by a liquid crystal display apparatus, a plasma display panel, a Cathode Ray Tube (CRT) display, or an Electroluminescence (EL) panel. The display unit 25 may have a voice output unit to output voices and sounds. The operation unit 26 includes a variety of operation buttons, a touch panel and other equivalent components, and accepts the various types of information from the user.

The communication unit 23 receives a response current value and activity factor data from the transmitting apparatus 1 via an antenna 24, and sends the received response current value and activity factor data to the control unit 21. The control unit 21 stores the received response current value and activity factor data in the storage unit 22. The control unit 21 calculates the glucose concentration value in the interstitial fluid from the response current value by using calibration curve data for calculating the glucose concentration value in the interstitial fluid from the response current value. The response current value is one example of a "signal". The control unit 21 is one example of a "calculation unit". The calibration curve data is one example of "arithmetic information". Plural items of calibration curve data are pre-stored in the storage unit 22. The calibration curve data represent a correspondence relation between the response current value and the glucose concentration value in the interstitial fluid. The calibration curve data are pre-stored as, e.g., a mathematical expression and a correspondence table in the storage unit 22.

For example, when the transmitting apparatus 1 is attached to a brachial region of the user, the measurement sensor 10 is indwelled subcutaneously in the brachial region of the user. For instance, when the transmitting apparatus 1 is attached to an abdominal region of the user, the measurement sensor 10 is indwelled subcutaneously in the abdominal region of the user. A value of the activity factor data when the measurement sensor 10 is subcutaneously in the brachial region of the user is different from a value of the activity factor data when the measurement sensor 10 is subcutaneously in the abdominal region of the user. Thus, the value of the activity factor data is different in every setting position (which will hereinafter be termed a "sensor setting position") of the measurement sensor 10 indwelled in vivo in the user. FIG. 4 is a diagram (and graphs) illustrating the value of the activity factor data when the measurement sensor 10 is indwelled subcutaneously in the brachial region of the user, and the value of the activity factor data when the measurement sensor 10 is indwelled subcutaneously in the abdominal region of the user. A graph A in FIG. 4 indicates the value of the activity factor data when the measurement sensor 10 is indwelled subcutaneously in the brachial region (notated as a "position A" in FIG. 4) of the user. A graph B in FIG. 4 indicates the value of the activity factor data when the measurement sensor 10 is indwelled subcutaneously in the abdominal region (notated as a "position B" in FIG. 4) of the user. As depicted in FIG. 4, the value of the activity factor data when the measurement sensor 10 is indwelled subcutaneously in the brachial region of the user is different from the value of the activity factor data when the measurement sensor 10 is indwelled subcutaneously in the abdominal region of the user.

Pre-stored in the storage unit 22 are the value of the activity factor data in every sensor setting position and information about the sensor setting position, which is associated with the value of the activity factor data. For example, when initializing the transmitting apparatus 1 and the receiving apparatus 2, the value of the activity factor data in every sensor setting position and the information about the sensor setting position, which is associated with the value of the activity factor data, may be transmitted to the receiving apparatus 2 from the transmitting apparatus 1 and stored in the storage unit 22. The information about the sensor setting position contains body regional information of the brachial region, the abdominal region, a gluteal region, leg regions and other equivalent regions. The control unit 21 compares the value of the activity factor data that is acquired from the transmitting apparatus 1 with the value of the activity factor data that is pre-stored in the storage unit 22. The control unit 21 identifies (determines) the sensor setting position, based on the activity factor data value acquired from the transmitting apparatus 1. The control unit 21 may identify the setting position of the detection sensor 17 with respect to the user on the basis of the activity factor data value acquired from the transmitting apparatus 1. The control unit 21 may also identify the setting position of the transmitting apparatus 1 with respect to the user on the basis of the activity factor data value acquired from the transmitting apparatus 1. The control unit 21 is one example of an "identifying unit".

The activity factor data contains the motion quantity data, in which case the control unit 21 identifies the sensor setting position, based on a value of the motion quantity data. To be specific, the control unit 21 identifies the sensor setting position, based on a motion quantity of the measurement sensor 10 or a motion quantity of the region of the user. For example, a motion quantity of the brachial region of the user per unit time is different from a motion quantity of the abdominal region of the user per unit time. Consequently, a value of the motion quantity data when the transmitting apparatus 1 is attached to the brachial region of the user, is different from a value of the motion quantity data when the transmitting apparatus 1 is attached to the abdominal region of the user. The control unit 21 compares the motion quantity data value acquired from the transmitting apparatus 1 with the motion quantity data value pre-stored in the storage unit 22, thereby identifying the sensor setting position. Though describing herein the example that the detection sensor 17 detects the motion quantity of the measurement sensor 10 or the motion quantity of the region of the user, the first embodiment is not limited to this example. The detection sensor 17 may also detect the motion quantity of the transmitting apparatus 1 or the motion quantity of the detection sensor 17. The control unit 21 may also identify the sensor setting position on the basis of the motion quantity of the transmitting apparatus 1 or the motion quantity of the detection sensor 17.

When the activity factor data contains attitude data, the control unit 21 identifies the sensor setting position, based on the attitude data. To be specific, the control unit 21 identifies the sensor setting position, based on an attitude of the measurement sensor 10 or an attitude of the region of the user. For example, the brachial region of the user has a tendency of taking a variety of attitudes, while the abdominal region of the user has a tendency of keeping the same attitude for a fixed period of time. The value of the attitude data when the transmitting apparatus 1 is set on the brachial region of the user is therefore different from the value of the attitude data when the transmitting apparatus 1 is set on the abdominal region of the user. The control unit 21 compares the attitude data value acquired from the transmitting apparatus 1 with the attitude data value pre-stored in the storage unit 22, thereby identifying the sensor setting position. Though describing herein the example that the detection sensor 17 detects the attitude of the measurement sensor 10 or the attitude of the region of the user, the first embodiment is not limited to this example. The detection sensor 17 may also detect the attitude of the transmitting apparatus 1 or the attitude of the detection sensor 17. The control unit 21 may also identify the sensor setting position on the basis of the attitude of the transmitting apparatus 1 or the attitude of the detection sensor 17.

When the activity factor data contains body temperature data, the control unit 21 identifies the sensor setting position on the basis of the body temperature data. In other words, the control unit 21 identifies the sensor setting position on the basis of the body temperature of the user. For example, a surface temperature of the brachial region of the user is different from a surface temperature of the abdominal region of the user. Hence, a value of the body temperature data when the detection sensor 17 is set on the brachial region of the user differs from a value of the body temperature data when the detection sensor 17 is set on the abdominal region of the user. The control unit 21 compares the body temperature data value acquired from the transmitting apparatus 1 with the body temperature data value pre-stored in the storage unit 22, thereby identifying the sensor setting position.

When the activity factor data contains pulse rate data, the control unit 21 identifies the sensor setting position, based on the pulse rate data. To be specific, the control unit 21 identifies the sensor setting position on the basis of the pulse rate of the user. For instance, the pulse rate of the user when measured at the brachial region of the user differs from the pulse rate of the user when measured at the abdominal region of the user. Consequently, a value of the pulse rate data when the detection sensor 17 is set on the brachial region of the user is different from a value of the pulse rate data when the detection sensor 17 is set on the abdominal region of the user. The control unit 21 identifies the sensor setting position by comparing the pulse rate data value acquired from the transmitting apparatus 1 with the pulse rate data value pre-stored in the storage unit 22.

When the activity factor data contains heart rate data, the control unit 21 identifies the sensor setting position, based on the heart rate data. Specifically, the control unit 21 identifies the sensor setting position on the basis of the heart rate of the user. For instance, the heart rate of the user when measured at the brachial region of the user differs from the heart rate of the user when measured at the abdominal region of the user. Therefore, a value of the heart rate data when the detection sensor 17 is set on the brachial region of the user is different from a value of the heart rate data when the detection sensor 17 is set on the abdominal region of the user. The control unit 21 identifies the sensor setting position by comparing the heart rate data value acquired from the transmitting apparatus 1 with the heart rate data value pre-stored in the storage unit 22.

When the activity factor data contains pulse wave data, the control unit 21 identifies the sensor setting position, based on the pulse wave data. To be specific, the control unit 21 identifies the sensor setting position on the basis of a waveform of the pulse wave of the user. For example, the waveform of the pulse wave of the user when measured at the brachial region of the user differs from a waveform of the pulse wave of the user when measured at the abdominal region of the user. Consequently, a value of the pulse wave data when the detection sensor 17 is set on the brachial region of the user is different from a value of the pulse wave data when the detection sensor 17 is set on the abdominal region of the user. The control unit 21 identifies the sensor setting position by comparing the pulse wave data value acquired from the transmitting apparatus 1 with the pulse wave data value pre-stored in the storage unit 22. The control unit 21 may calculate the pulse rate of the user by frequency-analyzing the waveform of the user's pulse wave, and may also identify the sensor setting position on the basis of the pulse rate of the user.

When the activity factor data contains blood pressure value data, the control unit 21 identifies the sensor setting position, based on the blood pressure value data. To be specific, the control unit 21 identifies the sensor setting position on the basis of the blood pressure value of the user. For instance, the blood pressure value of the user when measured at the brachial region of the user differs from the blood pressure value of the user when measured at the abdominal region of the user. Consequently, a value of the blood pressure value data when the detection sensor 17 is set on the brachial region of the user is different from a value of the blood pressure value data when the detection sensor 17 is set on the abdominal region of the user. The control unit 21 compares the value of the blood pressure value data that is acquired from the transmitting apparatus 1 with the value of the blood pressure value data that is pre-stored in the storage unit 22, thereby identifying the sensor setting position.

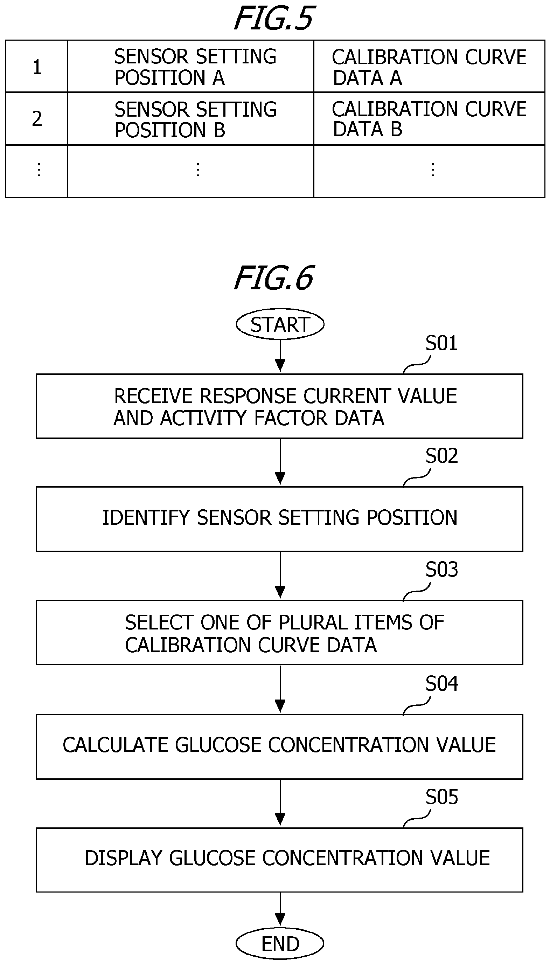

Plural items of calibration curve data and plural pieces of information about the sensor setting positions are pre-stored in the storage unit 22 in a state of associating the calibration curve data with the information about the sensor setting position. The control unit 21 selects one of the plural items of calibration curve data pre-stored in the storage unit 22, based on the sensor setting position. FIG. 5 is a table illustrating one example of the plural items of calibration curve data pre-stored in the storage unit 22. In the example illustrated in FIG. 5, a sensor setting position A is associated with calibration curve data A, and a sensor setting position B is associated with calibration curve data B. In FIG. 5, the calibration curve data A is different from the calibration curve data B, and, for example, an arithmetic parameter or another equivalent item of the calibration curve data A is different from the arithmetic parameter or another equivalent item of the calibration curve data B.

The control unit 21 calculates the glucose concentration value in the interstitial fluid on the basis of the response current value by using the selected calibration curve data. The selected calibration curve data is associated with the identified sensor setting position. Accordingly, the control unit 21 calculates the glucose concentration value in the interstitial fluid from the response current value with reference to the calibration curve data associated with the sensor setting position. Thus, the control unit 21 calculates the glucose concentration value in the interstitial fluid from the response current value with reference to the calibration curve data selected from the plural items of calibration curve data, which are each different for every sensor setting position.

FIG. 6 is a flowchart illustrating one example of a measuring process according to the first embodiment. After switching ON power sources of the transmitting apparatus 1 and the receiving apparatus 2, the transmitting apparatus 1 is fitted to the body surface of the user and is thus attached to the user. The transmitting apparatus 1 is initialized, and then the receiving apparatus 2 is initialized, thereby starting the flow illustrated in FIG. 6. The power source of the transmitting apparatus 1 may be switched ON after the transmitting apparatus 1 has been fitted to the body surface of the user. In step S01, the transmitting apparatus 1 transmits the response current value and the activity factor data to the receiving apparatus 2, and the receiving apparatus 2 receives the response current value and the activity factor data.

In step S02, the control unit 21 identifies the sensor setting position, based on the value of the activity factor data. In step S03, the control unit 21 selects one of the plural items of calibration curve data pre-stored in the storage unit 22, based on the sensor setting position. In step S04, the control unit 21 calculates the glucose concentration value in the interstitial fluid on the basis of the response current value by using the selected calibration curve data. In step S05, the display unit 25 displays the glucose concentration value in the interstitial fluid as the glucose concentration value of the user.

According to the first embodiment, the calibration curve data associated with the sensor setting position is selected from within the plural items of calibration curve data. The glucose concentration value in the interstitial fluid is calculated from the response current value by use of the selected calibration curve data. The glucose concentration value in the interstitial fluid is calculated from the response current value by using the calibration curve data selected from within the plural items of calibration curve data each being different for every sensor setting position, and measurement accuracy of the glucose concentration value in the interstitial fluid is thereby enabled to be improved.

### <Second Embodiment>

A measuring system according to a second embodiment will be described. The first embodiment has exemplified the example that one of the plural items of calibration curve data is selected based on the sensor setting position. The second embodiment will exemplify an example that one of plurality of measurement algorithms is selected based on the sensor setting position. The measurement algorithm is a processing procedure for measuring, e.g., the response current value. The measurement algorithm is one example of "arithmetic information". Note that the same components of the second embodiment as those of the first embodiment are marked with the same numerals and symbols as those of the first embodiment, and their repetitive explanations are omitted.

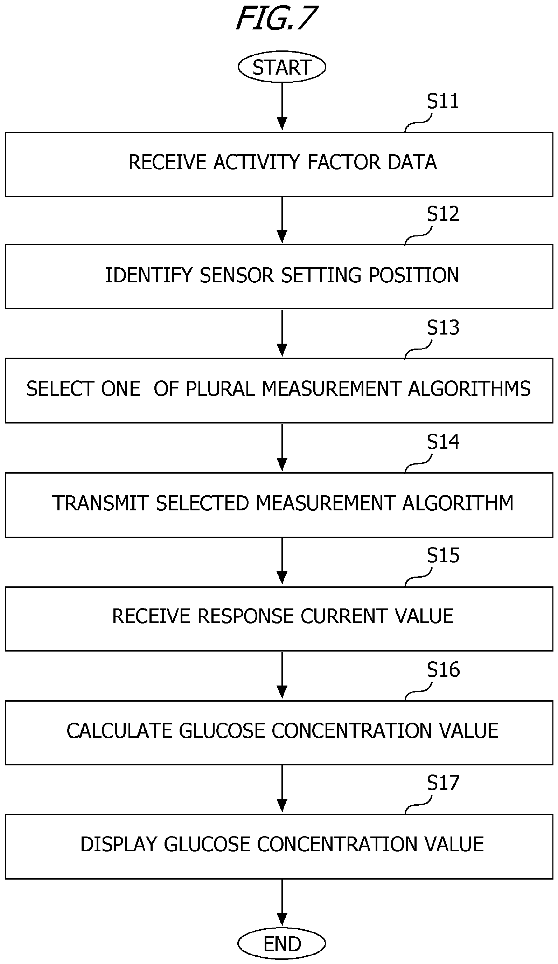

FIG. 7 is a flowchart illustrating one example of a measurement process according to the second embodiment. A condition for starting a flow illustrated in FIG. 7 is the same as the condition for starting the flow depicted in FIG. 6. In step S11, the transmitting apparatus 1 transmits the activity factor data to the receiving apparatus 2, and the receiving apparatus 2 receives the activity factor data. In step S12, the control unit 21 identifies the sensor setting position, based on the value of the activity factor data.

In step S13, the control unit 21 selects, based on the sensor setting position, one of plural measurement algorithms pre-stored in the storage unit 22. In a state of associating the measurement algorithm with information about the sensor setting position, the plurality of measurement algorithms and pieces of information about a plurality of sensor setting positions are pre-stored in the storage unit 22. FIG. 8 is a table illustrating one example of the plurality of measurement algorithms pre-stored in the storage unit 22. In the example illustrated in FIG. 8, a sensor setting position A is associated with a measurement algorithm A, and a sensor setting position B is associated with a measurement algorithm B. In Fig. 8, the measurement algorithm A is different from the measurement algorithm B, and, e.g., an applied voltage, a sampling interval, an arithmetic process and other equivalent items of the measurement algorithm A are different from the applied voltage, the sampling interval, the arithmetic process and other equivalent items of the measurement algorithm B.

In step S14, the receiving apparatus 2 transmits the selected measurement algorithm to the transmitting apparatus 1. The transmitting apparatus 1 receives the selected measurement algorithm. The control unit 13 controls a measurement unit 12 by using the selected measurement algorithm. The measurement unit 12 measures the response current value by applying a voltage to a sensor unit 11. The selected measurement algorithm is associated with the identified user setting position. Accordingly, the control unit 13 controls the measurement unit 12 by employing the measurement algorithm associated with the sensor setting position, and the measurement unit 12 measures the response current value. Thus, the control unit 13 controls the measurement unit 12 in a way that uses the measurement algorithm selected from within the plurality of measurement algorithms each being different for every sensor setting position, and the measurement unit 12 measures the response current value.

In step S15, the transmitting apparatus 1 transmits the response current value to the receiving apparatus 2, and the receiving apparatus 2 receives the response current value. In step S16, the control unit 21 calculates the glucose concentration value in the interstitial fluid on the basis of the response current value by employing predetermined calibration curve data. The predetermined calibration curve data is pre-stored in the storage unit 22. In step S17, the display unit 25 displays the glucose concentration value in the interstitial fluid as the glucose concentration value of the user.

In the state of associating the measurement algorithm with the information about the sensor setting position, the plurality of measurement algorithms and the pieces of information about the sensor setting positions may also be pre-stored in the storage unit 14. The control unit 13 of the transmitting apparatus 1 may select one of the plural measurement algorithms, based on the sensor setting position.

According to the second embodiment, the measurement algorithm associated with the sensor setting position is selected from within the plural measurement algorithms. The response current value is measured by using the selected measurement algorithm, and the glucose concentration value in the interstitial fluid is calculated from the response current value. The response current value is measured by use of the measurement algorithm selected from within the plurality of measurement algorithms each being different for every sensor setting position, and the glucose concentration value in the interstitial fluid is calculated from the response current value, thereby enabling improvement of the measurement accuracy of the glucose concentration value in the interstitial fluid.

In the first embodiment and the second embodiment, the control unit 21 identifies the sensor setting position. Without being limited to the examples demonstrated in the first embodiment and the second embodiment, the detection sensor 17 may identify the sensor setting position by providing a control circuit in the detection sensor 17. The detection sensor 17 is one example of an "identifying unit". The value of the activity factor data for every sensor setting position and the information about the sensor setting position associated with the value of the activity factor data, may also be pre-stored in the storage unit 14. For example, when initializing the transmitting apparatus 1 and the receiving apparatus 2, the value of the activity factor data for every sensor setting position and the information about the sensor setting position associated with the value of the activity factor data may be stored in the storage unit 14.

The measuring system according to the first embodiment may be combined with the measuring system according to the second embodiment. For example, the response current value is measured by using the measurement algorithm associated with the sensor setting position in the plurality of measurement algorithms, and the glucose concentration value in the interstitial fluid may be calculated from the response current value by use of the calibration curve data associated with the sensor setting position in the plural items of calibration curve data. The discussion made above has exemplified the measuring system including the transmitting apparatus 1 and the receiving apparatus 2, and each of the embodiments is not limited to this configuration. The transmitting apparatus 1 and the receiving apparatus 2 may be integrally configured as a measuring apparatus. Any one of the transmitting apparatus 1 and the receiving apparatus 2 may be configured as the measuring apparatus. The control unit 13 of the transmitting apparatus 1 may function as at least one of the "identifying unit" and the "calculation unit". The plural items of calibration curve data and the plurality of measurement algorithms may be stored in the storage unit 14 of the transmitting apparatus 1.

### <Third Embodiment>

A measuring system according to a third embodiment will be described. The same components of the third embodiment as those of the first and second embodiments are marked with the same numerals and symbols as those of the first and second embodiments, and their repetitive explanations are omitted.

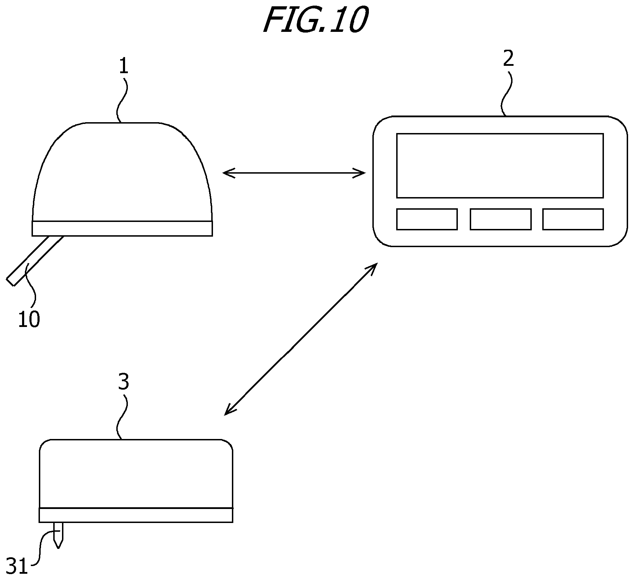

FIG. 10 is a diagram of a configuration of the measuring system according to the third embodiment. The measuring system illustrated in FIG. 10 includes the transmitting apparatus 1, the receiving apparatus 2 and a dosage apparatus 3. The transmitting apparatus 1 and the receiving apparatus 2 according to the third embodiment are the same as those of the first and second embodiments. The dosage apparatus 3 is a medicine supply apparatus to consecutively (continuously) or intermittently supply a medicine in vivo. The dosage apparatus 3 may be used by being attached to regions instanced by the abdominal region, the brachial region and the gluteal region. The dosage apparatus 3 may take any one of a patch (paste) type and a tube type. The medicines include insulin and glucagon. The dosage apparatus 3 performs wireless data communications with the receiving apparatus 2. The dosage apparatus 3 may perform wired data communications with the receiving apparatus 2. The transmitting apparatus 1 and the receiving apparatus 2, though configured as separate equipments in the third embodiment, may also be configured integrally. The transmitting apparatus 1 and the dosage apparatus 3, though configured as separate equipments in the third embodiment, may also be configured integrally. The transmitting apparatus 1, the receiving apparatus 2 and the dosage apparatus 3 may also be configured integrally.

### <Dosage Apparatus>

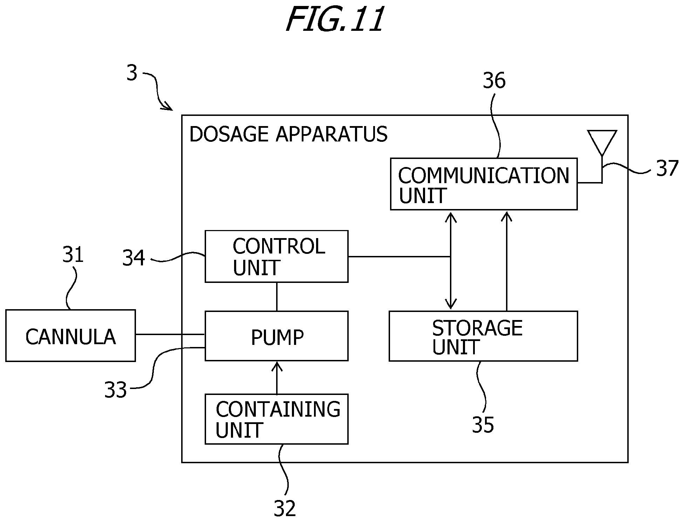

The dosage apparatus 3 includes a cannula (insertion unit) 31 used by being implanted into a subcutaneous region of the user. The dosage apparatus 3 is pasted to a skin of the user by an adhesive tape and other equivalent materials, or is attached to a piece of clothing, a belt and other equivalent articles, thereby being attached to the user. FIG. 11 is a block diagram of a configuration of the dosage apparatus 3 according to the third embodiment. The dosage apparatus 3 includes, the cannula 31, a containing unit 32, a pump 33, a control unit (arithmetic unit) 34, a storage unit 35, a communication unit 36, and an antenna 37. The cannula 31 is connected to the pump 33. The containing unit 32 contains medicines. The containing unit 32 may contain plural types of medicines. A plurality of containing units 32 may also be provided in the dosage apparatus 3. For example, one of the plural containing units 32 may contain insulin, while another of the plural containing units 32 may also contain glucagon.

The pump 33 is actuated by, e.g., a motor and other equivalent devices. The pump 33 is actuated to feed the medicine within the containing unit 32 to the cannula 31, whereby the medicine is dosed in vivo. The pump 33 is one example of a "dosage unit". A plurality of pumps 33 may be provided in the dosage apparatus 3. For instance, one of the plural pumps 33 may be an insulin pump, while another of the plural pumps 33 may also be a glucagon pump. The control unit 34 controls the pump 33, the storage unit 35 and the communication unit 36. The control unit 34 receives various items of data from the receiving apparatus 2 via the communication unit 36 and the antenna 37. The control unit 34 transmits the various items of data to the receiving apparatus 2 via the communication unit 36 and the antenna 37. The control unit 34, the storage unit 35 and the communication unit 36 may be attained by: computers each including the CPU, the RAM, the ROM and other equivalent hardware components that are provided in the dosage apparatus 3; respective apparatuses; and programs and other equivalent software components running on the computer.

The receiving apparatus 2 according to the third embodiment will be described. A plurality of dosage algorithms is pre-stored in the storage unit 22. The dosage algorithm is, e.g., a processing procedure for dosing the medicine in vivo. The dosage algorithm contains a type of the medicine to be dosed, a dose quantity (unit/min) of the medicine, a dose period of the medicine, and dose timing (dose time zone) of the medicine. The control unit 21 selects one of the plural dosage algorithms pre-stored in the storage unit 22 on the basis of the glucose concentration value in the interstitial fluid and the sensor setting position. In a state of associating the dosage algorithm, the glucose concentration value in the interstitial fluid and the information about the sensor setting position with each other, the plurality of dosage algorithms, the glucose concentration values in the interstitial fluids and pieces of information about the sensor setting positions, are pre-stored in the storage unit 22.

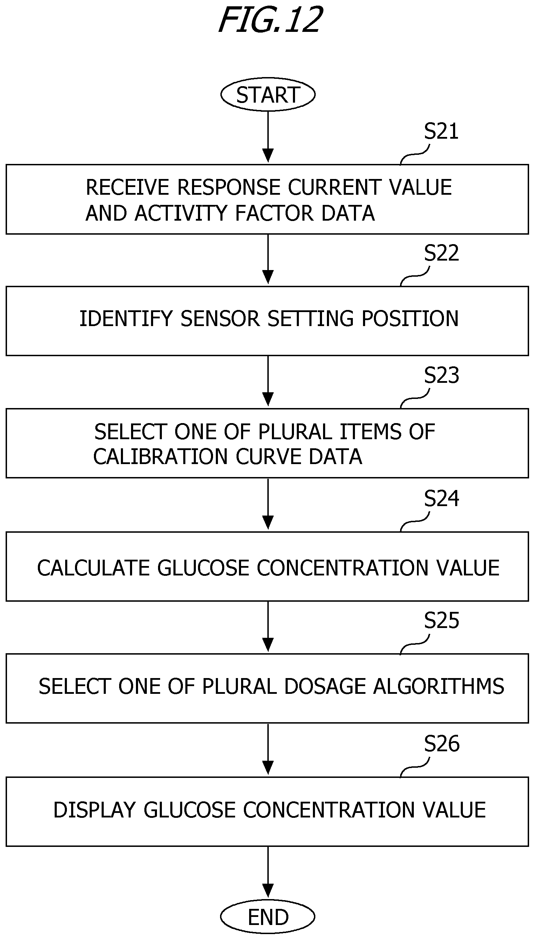

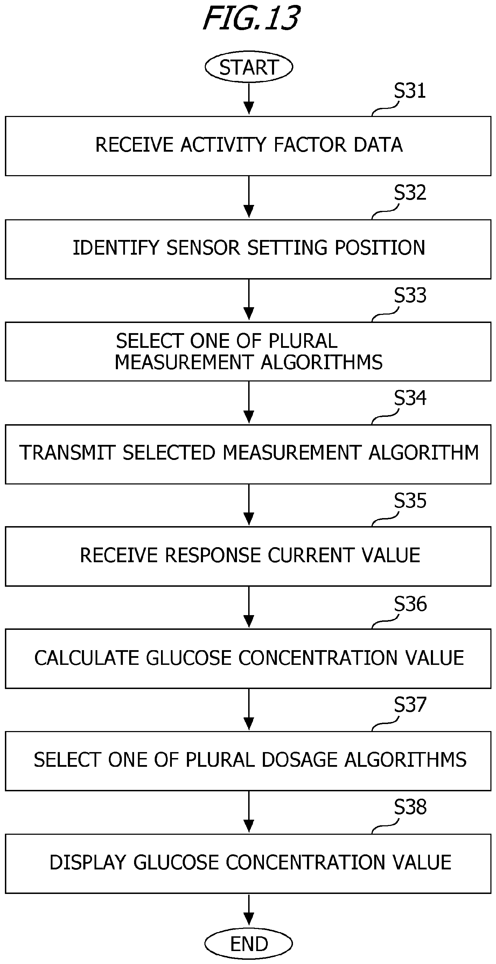

FIGS. 12 and 13 are flowcharts each illustrating a measurement process according to the third embodiment. Conditions for starting the flows illustrated in FIGS. 12 and 13 are the same as the condition for starting the flow illustrated in FIG. 6. Processes in steps S21 - S24 of FIG. 12 are the same as the processes in steps S01 - S04 of FIG. 6. Processes in steps S31 - S36 of FIG. 13 are the same as the processes in steps S11 - S16 of FIG. 7. In steps S25 and S37, the control unit 21 selects one of the plural dosage algorithms pre-stored in the storage unit 22, based on the glucose concentration value in the interstitial fluid and the sensor setting position. For example, insulin is selected as the type of the medicine to be dosed when the glucose concentration value in the interstitial fluid is equal to or larger than a first threshold value, and glucagon is selected as the type of the medicine to be dosed when the glucose concentration value in the interstitial fluid is less than the first threshold value. For instance, the insulin is selected as the type of the medicine to be dosed when the glucose concentration value in the interstitial fluid is equal to or larger than the first threshold value, and the glucagon is selected as the type of the medicine to be dosed when the glucose concentration value in the interstitial fluid is equal to or less than a second threshold value. The dose quantity of the medicine is determined corresponding to the glucose concentration value in the interstitial fluid and the sensor setting position. For example, the sensor setting position is different even when each of the glucose concentrations in the interstitial fluids takes the same value, in which case the dose quantity of the medicine differs. The dose period of the medicine may be determined corresponding to the sensor setting position. The dose timing of the medicine may also be determined corresponding to the sensor setting position.

In steps S26 and S38, the display unit 25 displays the glucose concentration value in the interstitial fluid as the glucose concentration value of the user. The process in step S25 is executed after the process in step S24 in the example of the flow in FIG. 12, and may also be executed after the process in step S26. The process in step S37 is executed after the process in step S36 in the example of the flow in FIG. 13, and may also be executed after the process in step S38.

After the glucose concentration value of the user has been displayed on the display unit 25, the control unit 21 controls the pump 33 so as to dose the medicine in vivo by using the selected dosage algorithm. For instance, the control unit 21 controls the pump 33 via the control unit 34 of the dosage apparatus 3. Before the glucose concentration value of the user is displayed on the display unit 25, the control unit 21 may control the pump 33 so as to dose the medicine in vivo by using the selected dosage algorithm. The pump 33 is actuated to feed the medicine within the containing unit 32 to the cannula 31, thereby dosing the medicine in vivo. Upon dosing the medicine in vivo, the glucose concentration value in vivo increases or decreases due to an effect of the medicine. The glucose concentration value in the blood and the glucose concentration value in the interstitial fluid are thereby increased or decreased. Upon dosing the insulin in vivo, the glucose concentration value in the blood and the glucose concentration value in the interstitial fluid decrease. Upon dosing the glucagon in vivo, the glucose concentration value in the blood and the glucose concentration value in the interstitial fluid increase.

The control unit 21 selects the dosage algorithm associated with the glucose concentration value in the interstitial fluid and the sensor setting position from within the plural dosage algorithms. The control unit 21 doses the medicine in vivo by controlling the actuation of the pump 33, based on the selected dosage algorithm. For instance, when the glucose concentration in the interstitial fluid has a high value, the insulin is dosed in vivo, whereby the glucose concentration value in the interstitial fluid decreases. For example, when the glucose concentration in the interstitial fluid has a low value, the glucagon is dosed in vivo, whereby the glucose concentration value in the interstitial fluid increases. The medicine is thus dosed in vivo, and the glucose concentration in the interstitial fluid is thereby enabled to keep a desired value.

A part or a whole of the processes executed by the control unit 21 of the receiving apparatus 2 may also be carried out by the control unit 34 of the dosage apparatus 3. For example, the control unit 34 receives the glucose concentration value in the interstitial fluid and the sensor setting position from the receiving apparatus 2, and may select one of the plural dosage algorithms pre-stored in the storage unit 35 on the basis of the glucose concentration value in the interstitial fluid and the sensor setting position. The control unit 34 may control the pump 33 so as to dose the medicine in vivo by using the selected dosage algorithm.

For example, a program may be stored in a memory of the computer equipped in at least one of the transmitting apparatus 1, the receiving apparatus 2 and the dosage apparatus 3, and may be run by the computer, whereby there respective processes in the first through third embodiments may also be attained. The computer may include a processor instanced by the CPU, a Micro Processing Unit (MPU) and a Field Programmable Gate Array (FPGA), and may also include a dedicated processor instanced by an Application Specific Integrated Circuit (ASIC). The respective processes in the first through third embodiments may be attained based on a method by which the computer runs the program. The program may be provided to the computer via, e.g., a network or from a computer readable recording medium and other equivalent mediums that retain the data in a non-transitory manner. The program may be recorded on the computer readable recording medium.

### <Computer Readable Recording Medium>

It is possible to record a program which causes a computer, machine, system (hereinafter, described as computer and other equivalent hardware components) to implement any of the functions described above on a computer readable recording medium. By causing the computer and other equivalent hardware components to read in the program from the recording medium and execute it, the function thereof can be provided. The computer readable recording medium mentioned herein indicates a recording medium which stores information such as data and a program by an electric, magnetic, optical, mechanical, or chemical operation and allows the stored information to be read from the computer and other equivalent hardware components. Of such recording media, those detachable from the computer or the like include, e.g., a flexible disk, a magneto-optical disk, a CD-ROM, a CD-R/W, a DVD, a Blu-ray disc, a DAT, an 8-mm tape, a flash memory and a memory card. Of such recording media, those fixed to the computer and other equivalent hardware components include a hard disk, a ROM or the like.

## Claims

1. A measuring apparatus comprising:
a sensor (10) configured to be indwelled, in use, in vivo of a user;
an identifying unit (13, 17, 21) configured to identify a setting position of the sensor (10);
a calculation unit (21) configured to calculate a concentration of a specified substance contained in a sample, based on a signal given from the sensor (10); and
a storage unit (22) configured to store plural pieces of arithmetic information,
wherein the calculation unit (21) selects one of the plural pieces of arithmetic information from the storage unit (22), based on the setting position of the sensor (10) identified by the identifying unit (13, 17, 21) and calculates the concentration of the specified substance contained in the sample by using the selected arithmetic information;
**characterized by** a detection sensor (17) configured to be attached to the user to detect an activity factor of the user, and in that the identifying unit (13, 17, 21) is configured to identify the setting position of the sensor (10) based on the activity factor of the user acquired from the detection sensor (17).

2. The measuring apparatus according to claim 1, wherein the plural pieces of arithmetic information contain plural items of calibration curve data, and
the calculation unit (21) selects one of the plural items of calibration curve data from the storage unit (22), based on the setting position of the sensor (10) identified by the identifying unit (13, 17, 21) and calculates the concentration of the specified substance contained in the sample by using the selected calibration curve data.

3. The measuring apparatus according to claim 1 or 2, wherein the plural pieces of arithmetic information contain plural measurement algorithms, and
the calculation unit (21) selects one of the plural measurement algorithms from the storage unit (22), based on the setting position of the sensor (10) identified by the identifying unit (13, 17, 21) and calculates the concentration of the specified substance contained in the sample by using the selected measurement algorithms.

4. The measuring apparatus according to any one of claims 1 through 3, wherein the activity factor comprises a motion quantity of the user.

5. The measuring apparatus according to any one of claims 1 through 4, wherein the activity factor comprises an attitude of the sensor.

6. The measuring apparatus according to any one of claims 1 through 5, wherein the activity factor comprises a body temperature of the user.

7. The measuring apparatus according to any one of claims 1 through 6, wherein activity factor comprises a pulse rate, a heart rate or a pulse wave in respect of the user.

8. The measuring apparatus according to any one of claims 1 through 7, wherein the activity factor comprises a blood pressure value in respect of the user.

9. The measuring apparatus according to any one of claims 1 through 8, wherein the sensor (10) consecutively measures the signals.

10. The measuring apparatus according to any one of claims 1 through 9, wherein the sample is interstitial fluid.

11. The measuring apparatus according to any one of claims 1 through 10, further comprising:
a control unit (34) configured to control a dosage unit (33) configured to dose a medicine in vivo of the user,
wherein the storage unit (22) stores plural dosage algorithms,
the control unit (21) selects one of the plural dosage algorithms from the storage unit (22), based on the concentration of the specified substance and the setting position of the sensor (10) identified by the identifying unit (13, 17, 21) and controls the dosage unit (33) to dose the medicine in vivo of the user by using the selected dosage algorithm.

12. A measuring program for causing the measuring apparatus according to any one of claims 1 through 11 to execute:
a process of identifying a setting position of a sensor (10) indwelled in vivo;
a process of selecting one piece of arithmetic information from a storage unit (22) configured to store plural pieces of arithmetic information, based on the setting position of the sensor (10); and
a process of calculating a concentration of a specified substance contained in a sample by using the selected arithmetic information, based on a signal given from the sensor (10);
**characterized in that** the process of identifying identifies the setting position of the sensor (10) based on an activity factor of the user acquired from a detection sensor (17) attached to the user to detect the activity factor of the user.

13. A measuring method performed with the measuring apparatus according to any one of claims 1 through 11 comprising:
a process of identifying a setting position of a sensor (10) indwelled in vivo of the user;
a process of selecting one piece of arithmetic information from a storage unit (22) configured to store plural pieces of arithmetic information, based on the setting position of the sensor (10); and
a process of calculating a concentration of a specified substance contained in a sample by using the selected arithmetic information, based on a signal given from the sensor (10);
**characterized by** detecting, using a detection sensor (17) attached to the user, an activity factor of the user and identifying, based on the activity factor of the user acquired from the detection sensor (17), the setting position of the sensor (10).

## Patentansprüche

1. Messvorrichtung, die Folgendes umfasst:
einen Sensor (10), der zum In-vivo-Einsetzen, beim Gebrauch, in einen Benutzer konfiguriert ist;
eine Identifizierungseinheit (13, 17, 21), die zum Identifizieren einer Einstellposition des Sensors (10) konfiguriert ist;
eine Berechnungseinheit (21), die zum Berechnen einer Konzentration einer in einer Probe enthaltenen spezifizierten Substanz auf der Basis eines vom Sensor (10) gegebenen Signals konfiguriert ist; und
eine Speichereinheit (22), die zum Speichern mehrerer arithmetischer Informationen konfiguriert ist,
wobei die Berechnungseinheit (21) eine der mehreren arithmetischen Informationen aus der Speichereinheit (22) auf der Basis der durch die Identifizierungseinheit (13, 17, 21) identifizierten Einstellposition des Sensors (10) auswählt und die Konzentration der in der Probe enthaltenen spezifizierten Substanz anhand der ausgewählten arithmetischen Informationen berechnet;
**gekennzeichnet durch** einen Detektionssensor (17), der zum Anbringen am Benutzer konfiguriert ist, um einen Aktivitätsfaktor des Benutzers zu detektieren, und **dadurch**, dass die Identifizierungseinheit (13, 17, 21) zum Identifizieren der Einstellposition des Sensors (10) auf der Basis des vom Detektionssensor (17) erfassten Aktivitätsfaktors des Benutzers konfiguriert ist.

2. Messvorrichtung nach Anspruch 1, wobei die mehreren arithmetischen Informationen mehrere Elemente von Kalibrationskurvendaten enthalten, und
die Berechnungseinheit (21) eines der mehreren Elemente von Kalibrationskurvendaten aus der Speichereinheit (22) auf der Basis der durch die Identifizierungseinheit (13, 17, 21) identifizierten Einstellposition des Sensors (10) auswählt und die Konzentration der in der Probe enthaltenen spezifizierten Substanz anhand der ausgewählten Kalibrationskurvendaten berechnet.

3. Messvorrichtung nach Anspruch 1 oder 2, wobei die mehreren arithmetischen Informationen mehrere Messalgorithmen enthalten, und
die Berechnungseinheit (21) einen der mehreren Messalgorithmen aus der Speichereinheit (22) auf der Basis der durch die Identifizierungseinheit (13, 17, 21) identifizierten Einstellposition des Sensors (10) auswählt und die Konzentration der in der Probe enthaltenen spezifizierten Substanz mit Hilfe der ausgewählten Messalgorithmen berechnet.

4. Messvorrichtung nach einem der Ansprüche 1 bis 3, wobei der Aktivitätsfaktor eine Bewegungsgröße des Benutzers umfasst.

5. Messvorrichtung nach einem der Ansprüche 1 bis 4, wobei der Aktivitätsfaktor eine Lage des Sensors umfasst.

6. Messvorrichtung nach einem der Ansprüche 1 bis 5, wobei der Aktivitätsfaktor eine Körpertemperatur des Benutzers umfasst.

7. Messvorrichtung nach einem der Ansprüche 1 bis 6, wobei der Aktivitätsfaktor eine Pulsfrequenz, eine Herzfrequenz oder eine Pulswelle in Bezug auf den Benutzer umfasst.

8. Messvorrichtung nach einem der Ansprüche 1 bis 7, wobei der Aktivitätsfaktor einen Blutdruckwert in Bezug auf den Benutzer umfasst.

9. Messvorrichtung nach einem der Ansprüche 1 bis 8, wobei der Sensor (10) die Signale fortlaufend misst.

10. Messvorrichtung nach einem der Ansprüche 1 bis 9, wobei die Probe interstitielle Flüssigkeit ist.

11. Messvorrichtung nach einem der Ansprüche 1 bis 10, die ferner Folgendes umfasst:
eine Steuereinheit (34), konfiguriert zum Steuern einer Dosiereinheit (33), die zum In-vivo-Dosieren eines Medikaments in dem Benutzer konfiguriert ist,
wobei die Speichereinheit (22) mehrere Dosierungsalgorithmen speichert,
die Steuereinheit (21) einen der mehreren Dosierungsalgorithmen aus der Speichereinheit (22) auf der Basis der Konzentration der spezifizierten Substanz und der durch die Identifizierungseinheit (13, 17, 21) identifizierten Einstellposition des Sensors (10) auswählt und die Dosierungseinheit (33) zum In-vivo-Dosieren des Medikaments in dem Benutzer mit Hilfe des ausgewählten Dosierungsalgorithmus steuert.

12. Messprogramm zum Bewirken, dass die Messvorrichtung nach einem der Ansprüche 1 bis 11 Folgendes ausführt:
einen Prozess des Identifizierens einer Einstellposition eines in vivo eingesetzten Sensors (10);
einen Prozess des Auswählens einer arithmetischen Information aus einer Speichereinheit (22), die zum Speichern mehrerer arithmetischer Informationen auf der Basis der Einstellposition des Sensors (10) konfiguriert ist; und
einen Prozess des Berechnens einer Konzentration einer in einer Probe enthaltenen spezifizierten Substanz anhand der ausgewählten arithmetischen Information auf der Basis eines von dem Sensor (10) gegebenen Signals;
**dadurch gekennzeichnet, dass** der Prozess des Identifizierens die Einstellposition des Sensors (10) auf der Basis eines Aktivitätsfaktors des Benutzers identifiziert, der von einem am Benutzer angebrachten Erfassungssensor (17) detektiert wird, um den Aktivitätsfaktor des Benutzers zu detektieren.

13. Messverfahren, durchgeführt mit der Messvorrichtung nach einem der Ansprüche 1 bis 11, das Folgendes beinhaltet:
einen Prozess des Identifizierens einer Einstellposition eines in vivo in den Benutzer eingesetzten Sensors (10);
einen Prozess des Auswählens einer arithmetischen Information aus einer Speichereinheit (22), die zum Speichern mehrerer arithmetischer Informationen auf der Basis der Einstellposition des Sensors (10) konfiguriert ist; und
einen Prozess des Berechnens einer Konzentration einer in einer Probe enthaltenen spezifizierten Substanz anhand der ausgewählten arithmetischen Information auf der Basis eines von dem Sensor (10) gegebenen Signals;
**gekennzeichnet durch** Detektieren, mit Hilfe eines am Benutzer angebrachten Detektionssensors (17), eines Aktivitätsfaktors des Benutzers und Identifizieren der Einstellposition des Sensors (10) auf der Basis des vom Detektionssensor (17) erfassten Aktivitätsfaktors des Benutzers.

## Revendications

1. Appareil de mesure comportant:
un capteur (10) configuré pour être installé à demeure, lors de l'utilisation, in vivo d'un utilisateur;
une unité d'identification (13, 17, 21) configurée pour identifier une position de pose du capteur (10);
une unité de calcul (21) configurée pour calculer une concentration d'une substance spécifiée contenue dans un échantillon, en se basant sur un signal donné en provenance du capteur (10); et
une unité de stockage (22) configurée pour stocker plusieurs éléments d'informations arithmétiques,
dans lequel l'unité de calcul (21) sélectionne l'un parmi lesdits plusieurs éléments d'informations arithmétiques en provenance de l'unité de stockage (22), en se basant sur la position de pose du capteur (10) qui a été identifiée par l'unité d'identification (13, 17, 21) et calcule la concentration de la substance spécifiée contenue dans l'échantillon en utilisant les informations arithmétiques sélectionnées;
**caractérisé par** un capteur de détection (17) configuré pour être attaché sur l'utilisateur pour détecter un facteur d'activité de l'utilisateur, et en ce que l'unité d'identification (13, 17, 21) est configurée pour identifier la position de pose du capteur (10) en se basant sur le facteur d'activité de l'utilisateur qui a été acquis par le capteur de détection (17).

2. Appareil de mesure selon la revendication 1, dans lequel lesdits plusieurs éléments d'informations arithmétiques contiennent plusieurs éléments de données de courbe d'étalonnage, et
l'unité de calcul (21) sélectionne l'un parmi lesdits plusieurs éléments de données de courbe d'étalonnage en provenance de l'unité de stockage (22), en se basant sur la position de pose du capteur (10) qui a été identifiée par l'unité d'identification (13, 17, 21) et calcule la concentration de la substance spécifiée contenue dans l'échantillon en utilisant les données de courbe d'étalonnage sélectionnées.

3. Appareil de mesure selon la revendication 1 ou la revendication 2, dans lequel lesdits plusieurs éléments d'informations arithmétiques contiennent plusieurs algorithmes de mesure, et
l'unité de calcul (21) sélectionne l'un parmi lesdits plusieurs algorithmes de mesure en provenance de l'unité de stockage (22), en se basant sur la position de pose du capteur (10) qui a été identifiée par l'unité d'identification (13, 17, 21) et calcule la concentration de la substance spécifiée contenue dans l'échantillon en utilisant les algorithmes de mesure sélectionnés.

4. Appareil de mesure selon l'une quelconque des revendications 1 à 3, dans lequel le facteur d'activité comporte une quantité de mouvement de l'utilisateur.

5. Appareil de mesure selon l'une quelconque des revendications 1 à 4, dans lequel le facteur d'activité comporte une orientation du capteur.

6. Appareil de mesure selon l'une quelconque des revendications 1 à 5, dans lequel le facteur d'activité comporte une température corporelle de l'utilisateur.

7. Appareil de mesure selon l'une quelconque des revendications 1 à 6, dans lequel le facteur d'activité comporte une fréquence du pouls, une fréquence cardiaque ou une onde de pression en ce qui concerne l'utilisateur.

8. Appareil de mesure selon l'une quelconque des revendications 1 à 7, dans lequel le facteur d'activité comporte une valeur de pression artérielle en ce qui concerne l'utilisateur.

9. Appareil de mesure selon l'une quelconque des revendications 1 à 8, dans lequel le capteur (10) mesure consécutivement les signaux.

10. Appareil de mesure selon l'une quelconque des revendications 1 à 9, dans lequel l'échantillon est un liquide interstitiel.

11. Appareil de mesure selon l'une quelconque des revendications 1 à 10, comportant par ailleurs:
une unité de commande (34) configurée pour commander une unité de dosage (33) configurée pour doser un médicament in vivo de l'utilisateur,
dans lequel l'unité de stockage (22) stocke plusieurs algorithmes de dosage,
l'unité de commande (21) sélectionne l'un parmi lesdits plusieurs algorithmes de dosage en provenance de l'unité de stockage (22), en se basant sur la concentration de la substance spécifiée et sur la position de pose du capteur (10) qui a été identifiée par l'unité d'identification (13, 17, 21) et commande l'unité de dosage (33) pour doser le médicament in vivo de l'utilisateur en utilisant l'algorithme de dosage sélectionné.

12. Programme de mesure servant à amener l'appareil de mesure selon l'une quelconque des revendications 1 à 11 à exécuter:
un processus consistant à identifier une position de pose d'un capteur (10) installé à demeure in vivo;
un processus consistant à sélectionner un élément d'informations arithmétiques en provenance d'une unité de stockage (22) configurée pour stocker plusieurs éléments d'informations arithmétiques, en se basant sur la position de pose du capteur (10); et
un processus consistant à calculer une concentration d'une substance spécifiée contenue dans un échantillon en utilisant les informations arithmétiques sélectionnées, en se basant sur un signal donné en provenance du capteur (10) ;
**caractérisé en ce que** le processus consistant à identifier identifie la position de pose du capteur (10) en se basant sur un facteur d'activité de l'utilisateur qui a été acquis par un capteur de détection (17) attaché sur l'utilisateur pour détecter le facteur d'activité de l'utilisateur.

13. Procédé de mesure effectué avec l'appareil de mesure selon l'une quelconque des revendications 1 à 11 comportant:
un processus consistant à identifier une position de pose d'un capteur (10) installé à demeure in vivo de l'utilisateur;
un processus consistant à sélectionner un élément d'informations arithmétiques en provenance d'une unité de stockage (22) configurée pour stocker plusieurs éléments d'informations arithmétiques, en se basant sur la position de pose du capteur (10); et
un processus consistant à calculer une concentration d'une substance spécifiée contenue dans un échantillon en utilisant les informations arithmétiques sélectionnées, en se basant sur un signal donné en provenance du capteur (10) ;
**caractérisé par** les étapes consistant à détecter, en utilisant un capteur de détection (17) attaché sur l'utilisateur, un facteur d'activité de l'utilisateur et à identifier, en se basant sur le facteur d'activité de l'utilisateur qui a été acquis par le capteur de détection (17), la position de pose du capteur (10).
